# EUROPEAN PATENT SPECIFICATION

(11) **EP 3 359 143 B1**
(45) Date of publication and mention of the grant of the patent: **23.07.2025**
(21) Application number: 16778062.6
(22) Date of filing: 07.10.2016
(51) Int. Cl.: A61K 9/70, A61K 31/485, A61K 47/12, A61K 47/14

(54) **A DEVICE FOR THE TRANSDERMAL DELIVERY OF BUPRENORPHINE**
VORRICHTUNG ZUR TRANSDERMALEN FREISETZUNG VON BUPRENORPHIN
DISPOSITIF D'ADMINISTRATION TRANSDERMIQUE DE BUPRÉNORPHINE

(30) Priority: 08.10.2015 AR 10325315
(43) Date of publication of application: 15.08.2018
(73) Proprietor: Amarin Technologies S.A., Ciudad Autónoma de Buenos Aires 1416 (AR)
(72) Inventor: SCASSO, Alejandro Fabio, Province of Buenos Aires (AR); STEFANO, Francisco José Evaristo, City of Buenos Aires 1425 (AR)
(74) Representative: Mewburn Ellis LLP
(86) International application number: PCT/EP2016/074072
(87) International publication number: WO 2017/060472

(56) References cited:
- EP-A1- 2 810 646
- US-A1- 2010 119 585
- US-A1- 2013 331 803

## Description

The present invention refers to a device for the transdermal administration of Buprenorphine, which comprises an adhesive matrix, a backing layer and release liner, where said adhesive matrix comprises a non-cross-linked adhesive acrylic polymer and the polymer(s) of the adhesive matrix is/are without carboxyl functionality, Buprenorphine Base, Levulinic Acid and Isopropyl Myristate.

### BACKGROUND OF THE INVENTION

Buprenorphine is a partial opioid agonist, which acts at the level of the mu opioid receptor and that, also, has activity on the kappa opioid receptor. Buprenorphine can be administered through the intramuscular, intravenous, sublingual, buccal (film on buccal mucosa) and transdermal route, in this last case, by means of transdermal administration devices, also named as patches. It possesses an analgesic activity superior to morphine ((0.2 - 0.6 mg IM of Buprenorphine, equivalent to 5 - 15 mg IM of morphine). The comparison between oral morphine versus transdermal Buprenorphine is 1:110. (Sittl R, Likar R, Poulsen B. Equipotent doses of transdermal fentanyl and transdermal Buprenorphine in patients with cancer and non cancer pain: results of a retrospective cohort study. Clin. Ther. 2005; 27 (2): 225-237).

Transdermal Buprenorphine is administered by means of a patch applied onto the skin, which is usually applied once every 4-7 days. In Argentina, it is a product approved, under the commercial name of Restiva^{®}, which is commercialized in the U.S.A. as BuTrans^{®} and, in Europe as Norspan^{®}. This product is a device for transdermal administration of Buprenorphine -also named, indistinctly, Transdermal Delivery System or TDS- recommended for the treatment of moderate to severe pain that do not properly respond to non-opioid analgesics. The plasmatic steady state is reached during the first application. After patch removal, the buprenorphine concentrations declines, decreasing about 50% in 12 hours (10-24 hours range).

Transdermal delivery is one of the routes of drug administration more accepted, from the commercial perspective (Tacharodi, D. y Paduranga, R., Biomaterials, 16, 145 (1995)). By means of transdermal delivery systems, it is possible to obtain systemic effects, avoiding the effect of first hepatic pass metabolism (one of the main disadvantages of the orally administered products). Likewise they allow a controlled drugs delivery to the bloodstream, without the need of turning to invasive options, such as the intravenous or the intramuscular routes.

However, the same TDSs' nature implies that, after the use of the device, certain drug quantity remains in it. This remnant is susceptible to abuse, particularly in the case of drugs with high risk of abuse such as the opioids. For this reason, minimizing the drug content in the TDS is highly important.

Mainly, there are two ways to reduce the amount of drug in the TDS: diminishing the concentration of the drug in the adhesive matrix or reducing the TDS's size (active surface).

Reducing the TDS's size has also an additional advantage: greater comfort for the patient, given by the smallest size of the device, along with a more attractive aesthetical appearance, by offering a greater discretion during its use. However, diminishing the concentration of the drug contained in the adhesive matrix and/or reducing the size (the active surface) of the TDS cause, in consequence, the reduction of the amount of drug delivered.

Thus, it would be highly convenient and desirable to get a TDS that delivers therapeutic quantities of Buprenorphine, such as for example, the ones delivered by the 7-day Restiva^{®} or BuTrans^{®} patches, but with a smaller patch size and/or a lower content of active drug than this one.

### BRIEF DESCRIPTION OF THE INVENTION

The present invention refers to a device for transdermal administration of Buprenorphine, which includes an adhesive matrix layer, a backing layer and a release layer, where the adhesive matrix includes a non-crosslinked acrylic adhesive polymer and the polymer(s) of the adhesive matrix is/are without carboxyl functionality, Buprenorphine Base, Levulinic Acid and Isopropyl Myristate, where the concentration of Buprenorphine Base is equal or lower than 8%, the concentration of Levulinic acid is from about 5% to about 10%, and the Isopropyl Myristate concentration is from about 5% to about 10% with respect of the total dry weight of the adhesive matrix. The devices have a total surface lower than or equal to 23 cm².

### BRIEF DESCRIPTION OF THE FIGURES

**FIGURE 1****.** *Restiva*^{®} and formulation of Example 2 (reference) permeation profiles.
**FIGURE 2****.** *Restiva*^{®} and formulation of Example 2 (reference) plasmatic levels.
**FIGURE 3****.** *Restiva*^{®} and formulation of Example 2 (reference), normalized by AUC, plasmatic profiles comparison.
**FIGURE 4****.** Formulations of Examples 3 (reference) and 4 (reference) permeation profiles.
**FIGURE 5****.** *Restiva*^{®} and formulations of Examples 3 (reference) and 5 to 7 permeation profiles.
**FIGURE 6****.** *Restiva*^{®} and formulations of Examples 4 (reference) and 7, normalized, permeation profiles.
**FIGURE 7****.** *Restiva*^{®} and formulations of Examples 8 (reference) and 11 (reference), normalized, permeation profiles.
**FIGURE 8****.** *Restiva*^{®} and formulations of Example 13 permeation profiles.
**FIGURE 9****.** *Restiva*^{®} and formulation of Example 13 plasmatic profiles.
**FIGURE 10****.** *Restiva*^{®} and Example 13, normalized by AUC, plasmatic profiles comparison.

### DETAILED DESCRIPTION OF THE INVENTION

Taking into account that the reduction of the active surface of a TDS has, as a consequence, the decrease of the quantity of the administered active drug substance and, therefore, the daily doses, it is an object of the present invention, to get a TDS distinguished by the delivery of Buprenorphine per unit of surface that is significantly greater than the reference product, allowing the reduction of the patch size but keeping the same drug delivered rates.

Additionally, taking into account that the decrease of the concentration of active drug in a TDS has as a consequence the decrease of the release rate of the active substance, it is an aim of the present invention to get a TDS characterised by comprising a concentration of Buprenorphine lower than the reference product, but showing a Buprenorphine release rate per unit of area equal to or greater than that of the reference product.

Another aim of the present invention is to provide a TDS bioequivalent to the product commercialised as Restiva^{®}, BuTrans^{®} or Norspan^{®} but comprising a lower load of Buprenorphine of said reference product.

Another aim of the present invention is to provide a TDS bioequivalent to the product commercialised as Restiva^{®}, BuTrans^{®} or Norspan^{®} but showing an active surface lower than such said reference product Norspan^{®} but containing a lower load of Buprenorphine.

On the other hand, the relationship between the active drug load that comprise a TDS and the amount of said drug that is effectively released during its use, is what it is known as TDS "efficiency" of the TDS. Thus, a device characterized by a greater efficiency implies that even containing a lower active drug load, it effectively releases the quantity of active required to be therapeutically effective. It is, therefore, another aim of the present invention to provide a TDS more efficient than the reference product.

**Patent** EP2810646A1 "Transdermal delivery system" describes an invention that relates to a transdermal therapeutic system for the transdermal administration of buprenorphine, comprising a buprenorphine-containing selfadhesive layer structure comprising A) a buprenorphine-impermeable backing layer, and B) a buprenorphine-containing pressure-sensitive adhesive layer on said buprenorphine-impermeable backing layer, the adhesive layer comprising a) at least one polymer-based pressure-sensitive adhesive, b) an analgesically effective amount of buprenorphine base or a pharmaceutically acceptable salt thereof, c) a viscosity-increasing substance in an amount of about 0.1% to about 8% of said buprenorphine-containing pressure-sensitive adhesive layer, and d) a carboxylic acid selected from the group consisting of oleic acid, linoleic acid, linolenic acid, levulinic acid and mixtures thereof, in an amount sufficient so that said analgesically effective amount of buprenorphine is solubilized therein to form a mixture including said viscositiy-increasing substance, and wherein the carboxylic acid-, buprenorphine- and viscosity-increasing substance-containing mixture forms dispersed deposits in the said pressure-sensitive adhesive, and wherein said buprenorphine-containing pressure-sensitive adhesive layer is the skin contact layer.

**Patent** US2010/119585A1 "TRANSDERMAL THERAPEUTIC SYSTEM FOR ADMINISTERING THE ACTIVE SUBSTANCE BUPRENORPHINE" describes an invention that relates to a transdermal therapeutic system for administering the active substance buprenorphine. Said system comprises at least one carboxylic acid that determines the solubility of buprenorphine in the matrix layer and that can likewise be absorbed. The transdermal therapeutic system according to the invention is used in the treatment of pain and is characterized by a considerably increased utilization of the active substance.

**Patent** US2013/331803A1 "TRANSDERMAL THERAPEUTIC SYSTEM COMPRISING BUPRENORPHINE" is concerned with a TDS (called a transdermal therapeutic system or TTS therein) comprising buprenorphine and a method of manufacturing such a TDS. The TDS is used for the transdermal administration of buprenorphine and analogues thereof. In particular, the invention of the cited patent relates to the use of a TDS for analgesic purposes. The TDS according to the cited patent comprises a transdermal drug delivery composition comprising buprenorphine and an adhesive component, which is a mixture of a crosslinked and a non-crosslinked acrylic polymer and a penetration enhancer comprising a keto acid.

US Patent 6,264,980 "Transdermal resorption of active substances from supercooled masses of levulinic acid" describes the use of Levulinic acid as an auxiliary agent to increase the absorption of Buprenorphine by the skin. According to the teachings in such patent, is how it is formulated the reference product Restiva^{®}, which comprises Levulinic acid in an adhesive matrix based on a cross-linked adhesive acrylic polymer, with carboxyl functionality.

In contrast, according to the present invention, a TDS is provided for the administration of Buprenorphine that uses an adhesive matrix compounded by a non-crosslinked adhesive acrylic polymer and the polymer(s) of the adhesive matrix is/are without carboxylic functionality. A non-crosslinked adhesive acrylic polymer is used, as the crosslinked agents could interact with the drug, affecting stability and, therefore, the quality of the final product. Likewise, polymers without carboxylic functionality are used, due to the fact that Buprenorphine is quite soluble in polymers with carboxylic functionality, thus its use would require high concentrations of drug to reach the saturation of the system and get a proper active drug release.

Nevertheless, in an attempt to obtain a TDS for the release of Buprenorphine that uses an adhesive matrix that comprises a non-crosslinked adhesive polymer and the polymer(s) of the adhesive matrix is/are without carboxylic functionality and, as auxiliary agent, Levulinic acid, it was found that the developed formulations did not show the same release profile as the one obtained for the reference product.

In fact, while by the addition of Levulinic acid to the formulation it was obtained an increase in the Buprenorphine permeation through the skin, it was observed, as well, that the maximum drug flux was reached after a longer time of postapplication of the TDS (see formulations of examples 1 (reference) and 2 (reference)). This would imply that, by the use of this formulation, the plasma concentration needed to get a therapeutic effect of the drug would be reached later - it means that there would be a delay in the beginning of the therapeutic effect of the Buprenorphine - than with the reference product.

Thus, there is a need to be able to get a TDS that shows the advantages of the use of an adhesive matrix which comprises a non-crosslinked adhesive acrylic polymer and the polymer(s) of the adhesive matrix is/are without carboxylic functionality and Levulinic acid as auxiliary agent which, besides, allows getting a permeation profile similar to the reference product and, even more preferably, bioequivalent with the latter. So, in particular manner, there is a need to be able to accelerate the release of the drug from the TDS adhesive matrix for the administration of Buprenorphine, which includes a non-crosslinked adhesive acrylic polymer and the polymer(s) of the adhesive matrix is/are without carboxylic functionality and Levulinic acid as auxiliary agent.

Surprisingly, the inventors of the present invention have found that the addition of Isopropyl Myristate (IPM) to a TDS for the release of Buprenorphine, which uses an adhesive matrix that comprises a non-crosslinked adhesive acrylic polymer and the polymer(s) of the adhesive matrix is/are without carboxylic functionality and Levulinic acid as auxiliary agent, allows to get a TDS with an active surface smaller to the one from Restiva^{®}, but that provides a permeation profile similar to this last one.

Furthermore, the inventors of the present invention have surprisingly found that the addition of Isopropyl Myristate to a TDS for the release of Buprenorphine, which uses an adhesive matrix that includes a non-crosslinked adhesive acrylic polymer and the polymer(s) of the adhesive matrix is/are without carboxylic functionality and, as auxiliary agent, Levulinic acid, allows the obtention of a TDS in which the quantity of drug per unit of area is substantially greater than the one with Restiva^{®}. In other words, the addition of Isopropyl Myristate allows the obtention of a more efficient TDS for the administration of Buprenorphine.

Therefore, it is an aim of the present invention, a device for the transdermal administration of Buprenorphine, which comprises an adhesive matrix, a backing layer and a release liner, in which that adhesive matrix comprises a non-crosslinked adhesive acrylic polymer and the polymer(s) of the adhesive matrix is/are without carboxylic functionality, Buprenorphine Base, Levulinic acid and Isopropyl Myristate, where the Buprenorphine Base is found in a concentration less than or equal to 8% by weight of the total dry weight of said adhesive matrix, where the Levulinic acid is found in a concentration from about 5% to about 10% by weight of the total dry weight of said adhesive matrix and where the Isopropyl Myristate is found in a concentration from about 5% to about 10% regarding the total dry weight of the adhesive matrix. Such device preferably has a total surface smaller than or equal to 20 cm².

In a particular embodiment of the invention, a device for the transdermal administration of Buprenorphine of the above mentioned characteristics, where such adhesive matrix includes a non-crosslinked adhesive acrylic polymer and the polymer(s) of the adhesive matrix is/are without carboxylic functionality, which, preferably, is a polymer with hydroxyl functionality. In other particular embodiment, such non-crosslinked adhesive acrylic polymer, without carboxylic functionality, is a non-functional polymer. In a specially preferred embodiment, such non-crosslinked adhesive acrylic polymer, without carboxylic functionality is selected from the adhesives Duro-Tak 87-900A, 87-901A, 87-9301, 87-4098, 87-9088, 87-2510, 87-208A, 87-2287 and 87-4287.

In a preferred embodiment of the invention, the device for the transdermal administration of Buprenorphine of the invention also comprises polyvinylpyrrolidone (PVP). In other specially preferred embodiment, the polyvinylpyrrolidone is found in a concentration between about 5% and about 10% related to the total dry weight of the adhesive matrix. In other embodiment still more preferable, the polyvinylpyrrolidone is PVP K90.

In other preferred embodiment of the invention, the device of the invention for transdermal administration of Buprenorphine comprises, besides, a polyvinyl caprolactam, polyvinyl acetate and polyethylene glycol grafted copolymer. In another specially preferred embodiment, the polyvinyl caprolactam, polyvinyl acetate and polyethylene glycol grafted copolymer is found in a concentration from about 2.5% to about 10.0% regarding the total dry weight of the adhesive matrix.

In a specially preferred embodiment of the invention, the device for the transdermal administration of Buprenorphine comprises the adhesive polymer DT 87-4098 and comprises a concentration of about 8% of Buprenorphine Base, a concentration of about 10% of PVP, a concentration of about 10% of Levulinic acid and a concentration of about 10% of Isopropyl Myristate regarding the total dry weight of the adhesive matrix and, also, has a total surface of about 20 cm².

According to the present invention, the expressions "device for the transdermal administration", "transdermal administration system", "transdermal administration patch," and "TDS," unless otherwise indicated, are interchangeably used and should be considered as equivalents.

Likewise, according to the present invention, expressions like "Restiva^{®}", "BuTrans^{®}," "Norspan^{®}" and "reference product", unless otherwise indicated, are interchangeably used and should be considered as equivalents.

On the other hand, according to the present invention, terms like "drug", "active substance," "therapeutically active substance" are used indistinctly and should be considered as equivalents. Likewise, the term "Buprenorphine" covers this substance, as well as its salts and pharmaceutically acceptable derivates.

Thus, surprisingly, the present invention allows getting a product with a size and content of buprenorphine significantly smaller than those of the reference product, which implies that the product of the present invention presents, at the same time, more comfortable use for the patient. Likewise, the invention allows getting a product that, once the therapeutic use has concluded, has a minor remnant content of Buprenorphine after use than the reference product, and therefore it presents a lower risk once used, also reducing the possibility of abuse. Thus, it is another aim of the invention, a device for the transdermal administration that presents a lower risk of the drug remnant in it as regards to the reference product.

As can be appreciated from the examples included in the present invention, the effect observed in the permeation of Buprenorphine through the skin, when Levulinic acid and Isopropyl Myristate are combined in the formulation, is greater than the sum of the effects observed when the formulation contains either Levulinic acid or Isopropyl Myristate separately, which indicates that there is a non expected synergism between both components.

The main tool used by the inventors of the present invention to evaluate the permeation of Buprenorphine in the mentioned examples is *in vitro* comparative permeation experiments through human skin. The experiments were performed using Valia-Chen's diffusion cells, placing a portion of the TDS in contact with a probe of human skin, between the donor and receiver compartments of the cell. Regularly, aliquots from the receiver cell were extracted, which were analysed by means of an appropriate HPLC method.

The intrinsic variability related to the use of biological skin samples implies that absolute results obtained in different experiments are not directly comparable. For this reason, each experiment includes an evaluation of the reference product as control formulation.

There were used two ways of expressing the results of the permeation experiments flux for their analysis. The cumulative permeation represents the sum of the quantities of drug per unit of area found in all the samplings of the receiver compartment along the experiment. The drug flux through the skin represents the quantity of drug permeated through the skin per unit of area and time, for each sampling time.

The cumulative permeation allows the evaluation of the capability that a certain formulation has to administer a drug through the skin and the quantity reached at the end of the experiment is preferable evaluated. The flux of the drug as a function of time represents the permeation profile of the formulation and allows evaluating the rate to which the drug goes through the skin.

In the *In Vivo* studies described at the present invention, the area under the curve (AUC) of plasma profile of Buprenorphine is used as indicator of the amount of drug administered to the patient. At the time of estimating the size that should have a TDS to be able to administer the same amount of drug as is in a determined reference product, it is made a theoretical calculation of the size that should proportionally have such SAT to reach an AUC equal to the one shown by the reference.

Buprenorphine is a drug with a strong trend to crystallization, so that from certain concentrations, it usually crystallizes in the adhesive matrix where they are included. Crystallization can increase with time and create negative effects as regards TDS adhesion performance as well as drug release profile. For this, it is convenient to add a proper crystallization inhibitor. In particular embodiment, polyvinylpyrrolidone polymers are used as crystallization inhibitors, preferably with a K value between 17 and 90 and, more preferably still, with a K value of 90. In other particular embodiments, the crystallization inhibitor selected is a polyvinyl caprolactam, polyvinyl acetate and polyethylene glycol grafted copolymer.

Within the scope of the present invention, it is considered also to include other appropriate ingredients to the formulation for the use in TDS, such as antioxidants, other permeation promoters, tackifying agents, co-solvents, etc. The skilled person in the art will surely know to select the more appropriate ingredients and determine the proper concentration to be used. Within scope of the present invention, it is also considered the possibility to include other drugs which complement the therapeutic action of the Buprenorphine in the formulation.

Moreover, it is also considered, within the scope of the present recent invention, the possibility to include additional means to grant the adhesion of the TDS to the skin during the desired time of use. Said means may include, among other options, to add an additional matrix adhesive layer to the TDS, to be in contact with the patient's skin, to add an overlay larger than the drug-containing layer or other options known by the person skilled in the art.

In an especially preferred embodiment of the invention, the TDS comprises an adhesive overlay whose ends extend beyond the edges of the drug-containing adhesive matrix. In this embodiment, between the drug-containing adhesive layer and the overlay, a barrier film is placed that avoids the diffusion of the drug from the adhesive matrix containing the drug to the overlay.

In order to better describe the invention, several examples of embodiments are included hereafter. These examples are given for illustrative purposes only and in no way restrict the scope of the invention. In this way, the present invention comprises all and every variation which would become evident as a result of the teachings provided herein.

### EXAMPLES

The following examples show the efficiency of the invention described herein. Unless otherwise indicated, the concentrations of the ingredients in the different examples are shown as a percentage in weight related to the total dry weight of the adhesive matrix which includes the drug.

### Example 1 (reference)

### Comparison between the reference TDS (Restiva^{®}) and a TDS that does not include either Levulinic acid nor Isopropyl Myristate

**Table 1: Formulation of Example 1 (reference)**

| Ingredient | Example 1 (reference) (BPF 230) |
|---|---|
| Buprenorphine Base | 10.0 % |
| DT 87-4287 | 80.0 % |
| PVP K90 | 10.0 % |

An In-Vitro comparative permeation experiment through human skin testing the formulation of Example 1 (reference) and the patch of reference (Restiva^{®}) was performed, withdrawing samples regularly along 168 hours (7 days).

At the end of the experiment averaged quantity of Buprenorphine cumulated in the diffusion cell was 151 µg/cm² for Restiva^{®} and 95 µg/cm² for the formulation of Example 1 (reference), with a permeation of about 40% lower than the one of the reference product.

### Example 2 (reference)

### Levulinic acid effects in the permeation of Buprenorphine (In-Vitro results)

**Table 2: Formulation of Example 2 (reference)**

| Ingredient | Example 2 (reference) (BPF 218) |
|---|---|
| Buprenorphine Base | 10.0 % |
| DT 87-4287 | 70.0 % |
| PVP K90 | 10.0 % |
| Levulinic Acid | 10.0 % |

An *In-Vitro* comparative permeation experiment through human skin was performed utilizing TDS manufactured using the formulation of Example 2 (reference) and the patch of reference (Restiva^{®}), withdrawing samples regularly along 168 hours (7 days).

At the end of the experiment the average quantity of Buprenorphine cumulated in the diffusion cell was 134 µg/cm² for Restiva^{®} and 231 µg/cm² for the formulation of Example 2 (reference), with a permeation of about 70% greater than the one of the reference product. This result corresponds to expectations, regarding the previous art.

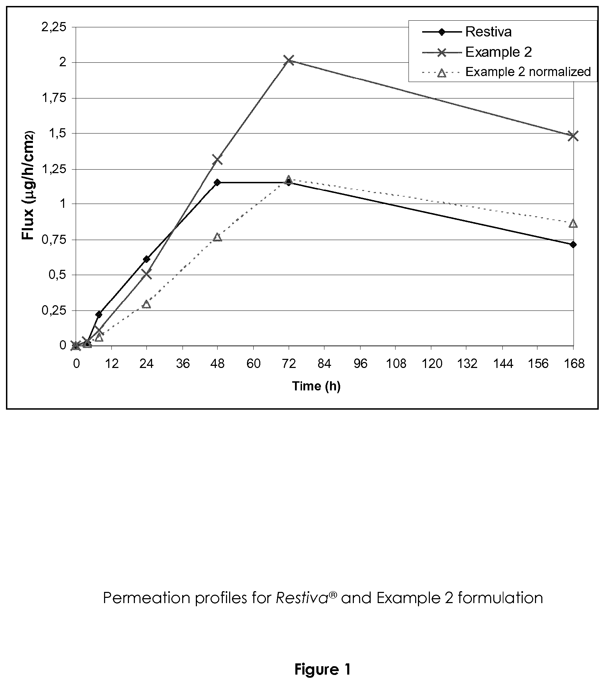

However, from Figure 1, important differences are shown in the permeation profile in Example 2 (reference) when compared with the one shown by Restiva^{®}.

By normalizing the results so that the same amount of Buprenorphine is cumulated after 168 hours for both, Example 2 (reference) and also Restiva^{®} (which is equivalent to reduce the size of the TDS so as to equal the daily dose with the reference product), It can be observed that the flux of Buprenorphine obtained with the formulation of Example 2 (reference) requires more time to reach maximum values than the one obtained with Restiva^{®}.

### Example 3 (reference)

### Levulinic acid effects in the permeation of Buprenorphine (In-Vitro results)

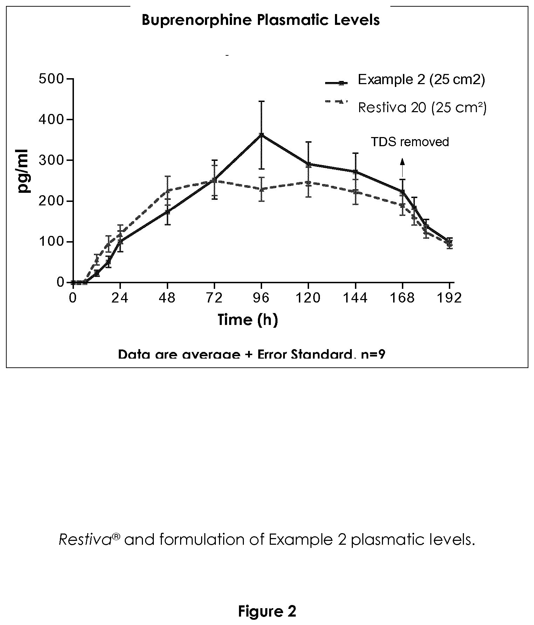

A pilot pharmacokinetics open randomised double crossover (3 periods and 3 sequences) study was carried out on nine (9) healthy individuals (volunteers) under in-patient conditions and Naltrexone block, in which the plasma levels reached by TDSs with the formulation of Example 2 (reference) and by Restiva^{®} were compared (both formulations with an active surface of 25 cm²). Results are shown in Figure 2.

The analysis of the results of the study suggests that a TDS with the formulation of Example 2 (reference)and an active surface of 22.5 cm² theoretically would show an AUC equal to Restiva^{®} with an active surface of 25 cm².

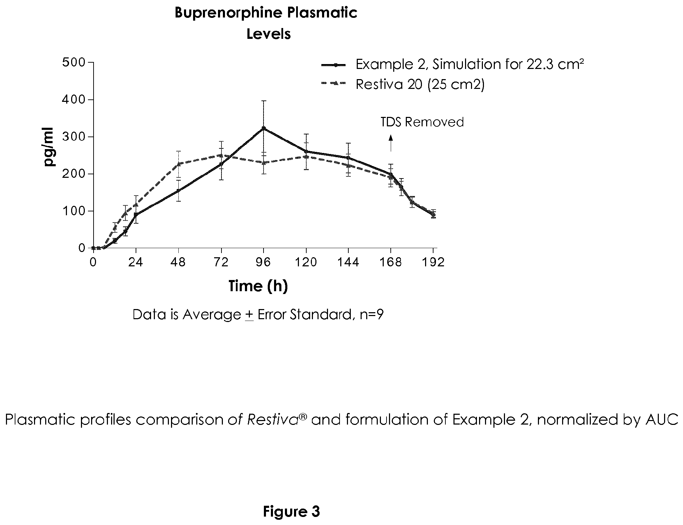

In Figure 3, a comparison is made between the simulation of the theoretical plasmatic profile reached by a TDS with the formulation of Example 2 (reference) and an active surface of 22.5 cm² and Restiva^{®} with an active surface of 25 cm².

The results obtained from the clinical study allow to qualitatively corroborate the results obtained in the *In-Vitro* permeation experiments, providing the formulation of Example 2 (reference) a Buprenorphine release greater but more delayed than the one of the reference product.

### Example 4 (reference)

### Addition of Isopropyl Myristate to the Levulinic Acid formulation

**Table 3 Example 3 (reference) and Example 4 (reference) formulations**

| **Ingredient** | **Example 3 (reference) (BPF 253)** | **Example 4 (reference) (LP 40458)** |
|---|---|---|
| Buprenorphine Base | 10.0 % | 10.0 % |
| DT 87-4287 | 60.0 % | 70.0 % |
| PVP K90 | 10.0 % | 10.0 % |
| Levulinic Acid | 10.0 % | 10.0 % |
| Isopropyl Myristate (MIP) | 10.0 % | --- |

An In-Vitro comparative permeation experiment through human skin during 168 hours, comparing Example 3 (reference) and Example 4 (reference) formulations was performed. After 168 hours of permeation, the average amount of Buprenorphine cumulated in the receiver cell was 225 µg/cm² for the formulation of Example 3 (reference), and 160 µg/cm² for formulation of Example 4 (reference).

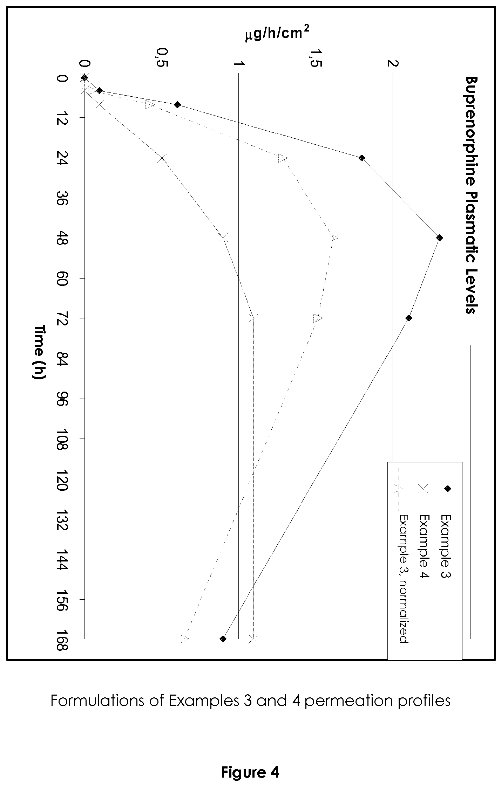

Figure 4 shows that the addition of Isopropyl Myristate not only increases significantly the permeation of Buprenorphine through the skin, but also modifies the drug permeation profile, reaching maximum levels of flux quicker than in the absence of Isopropyl Myristate.

### Examples 5, 6 and 7

### Effects of the concentration of Buprenorphine

**Table 4: Formulations of Example 3 (reference), 5 to 7**

| **Ingredient** | **Example 5 (BPF 269)** | **Example 6 (BPF 270)** | **Example 7 (BPF 264)** | **Example 3 (reference) (BPF 253)** |
|---|---|---|---|---|
| Buprenorphine Base | 4.0 | 6.0 | 8.0 | 10.0 |
| DT 87-4287 | 66.0 | 64.0 | 62.0 | 60.0 |
| PVP K90 | 10.0 | 10.0 | 10.0 | 10.0 |
| Levulinic acid | 10.0 | 10.0 | 10.0 | 10.0 |
| Isopropyl Myristate | 10.0 | 10.0 | 10.0 | 10.0 |

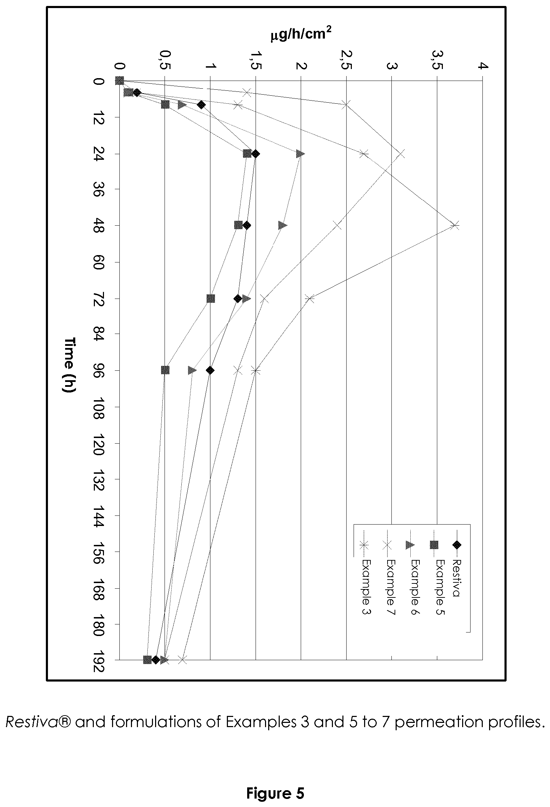

*In-Vitro* comparative permeation experiment was conducted of different formulations shown in Table 4, comparing the profiles of such formulations with Restiva^{®} TDSs (Figure 5), as well as the amounts of Buprenorphine cumulated after 192 hours reached by each formulation (Table 5).

**Table 5: Cumulative permeation results for Restiva^{®} and Example 3 (reference) and 5 to 7 after 192 hours**

| **Formulation** | **Cumulative amount of Buprenorphine (µg/cm²)** | **Percentage regarding *Restiva*^{®}** |
|---|---|---|
| *Restiva*^{®} | 155 | - |
| Example 5 | 119 | 76.8 % |
| Example 6 | 174 | 112% |
| Example 7 | 240 | 155 % |
| Example 3 (reference) | 289 | 186 % |

Figure 5 shows that the formulation of Example 7 represents the best compromise between obtaining a profile similar to that of Restiva^{®} and a significant increase of the Buprenorphine release.

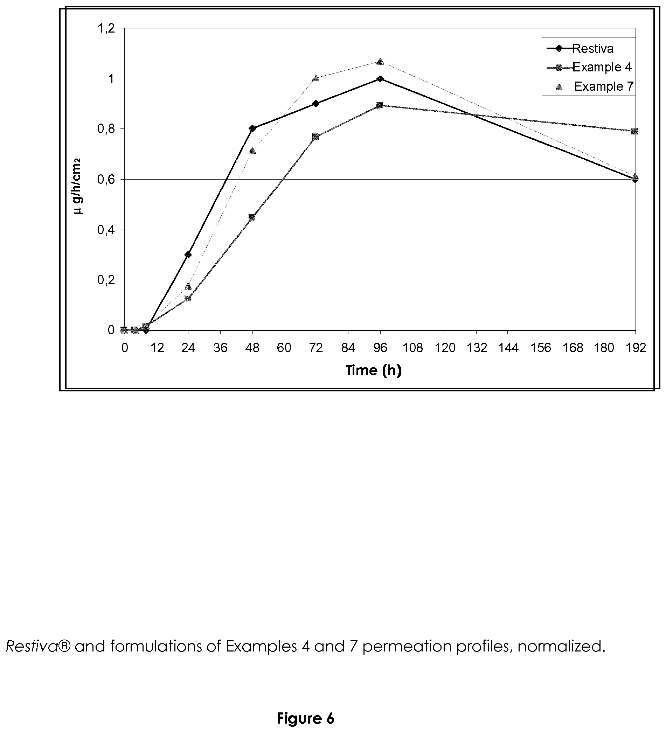

This observation is clearer in Figure 6. It includes results of a new comparative permeation experiment, which allows concluding that the formulation of Example 7 shows a permeation profile closer to that of the Restiva^{®} than to that of the formulation of Example 4 (reference).

### Example 8 (reference), Example 9 (reference), Example 10, and Example 11 (reference)

### Formulations with several concentrations of Isopropyl Myristate and Levulinic Acid

**Table 6: Formulation of Examples 8 to 11**

| **Ingredient** | **Example 8 (reference)** | **Example 9 (reference)** | **Example 10** | **Example 11 (reference)** |
|---|---|---|---|---|
| | | | **BPF 309** | |
| | **BPF 307** | **BPF 308** | | **BPF 320** |
| Buprenorphine Base | 8.00 | 8.00 | 8.00 | 8.00 |
| DT 87-4098 | 72.0 | 72.0 | 72.0 | 82.00 |
| PVP K90 | 10.0 | 10.0 | 10.0 | 10.00 |
| Levulinic Acid | 10.0 | --- | 5.00 | --- |
| Isopropyl Myristate | --- | 10.0 | 5.00 | --- |

An In-Vitro comparative permeation experiment was conducted through human skin during 168 hours, comparing Example 8 (reference), Example 9 (reference), Example 10, and Example 11 (reference) with TDS of Restiva^{®}. The formulations of these examples were prepared based on an adhesive without functionality.

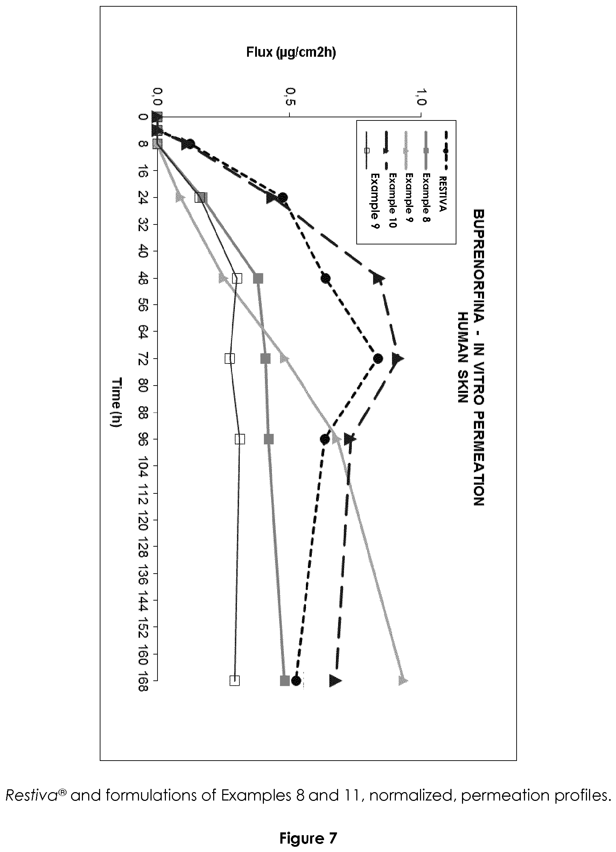

The profiles obtained were compared for formulations of the examples with the one obtained for Restiva^{®} (figure 7), likewise the cumulated quantities of Buprenorphine after 192 hours reached for each formulation (Table 7)

**Table 7: Cumulative permeation results for Restiva^{®} and the formulations of Examples 8 to 11, after 168 hours.**

| **Formulation** | **Cumulative amount of Buprenorphine (µg/cm²)** | **Percentage regarding *Restiva*^{®}** |
|---|---|---|
| Restiva^{®} | 96.5 | --- |
| Example 8 (reference) | 66.5 | 69 % |
| Example 9 (reference) | 102.6 | 106 % |
| Example 10 | 116.0 | 120% |
| Example 11 (reference) | 45.0 | 47% |

Figure 7 shows that, in the formulation of Example 8 (reference), the addition of 10% of Isopropyl Myristate increases very little the permeation flux with regards to the formulation of Example 11 (reference), which does not include Isopropyl Myristate or Levulinic Acid. For the formulation of Example 9 (reference), it shows a significant increase of the flux, but only after 72 hours. When in the formulation of Example 10 it is combined Isopropyl Myristate with Levulinic Acid, it does not only increase the flux of the drug which permeates, but this happens from the very beginning of the experiment, as it happens in the reference product.

### Example 12

### Formulation with Acrylic adhesive without functionality

**Table 8: Formulation of Example 13**

| **Ingredient** | **Example 13 (BPF 290)** |
|---|---|
| Buprenorphine Base | 8.0 |
| DT 87-4098 | 62.0 |
| PVP K90 | 10.0 |
| Levulinic acid | 10.0 |
| Isopropyl Myristate | 10.0 |

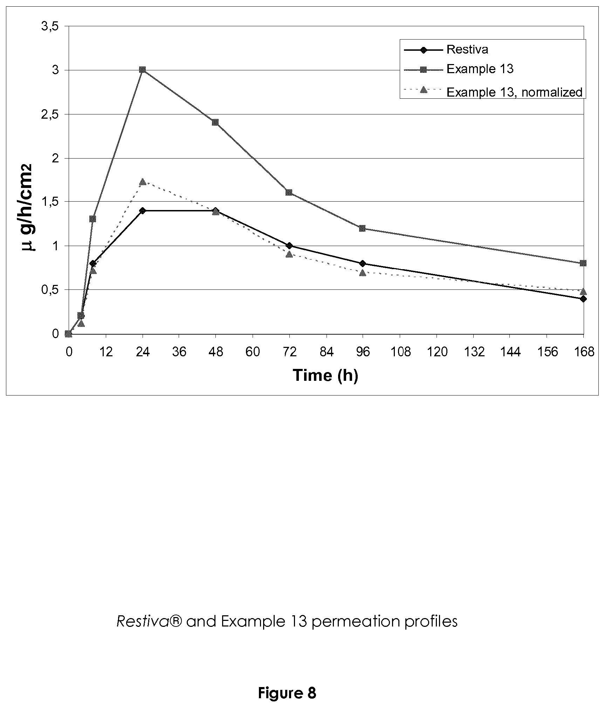

Figure 8 shows that the use of an acrylic adhesive polymer without functionality, as a base for the adhesive matrix, allows to obtain a TDS that meets the intended purposes. After 168 hours of permeation, the average cumulated amount of Buprenorphine in the receiver cell was 137 µg/cm² for Restiva^{®} and 240 µg/cm² for the formulation of Example 13.

### Example 13

### In Vivo evaluation of Example 13 formulation

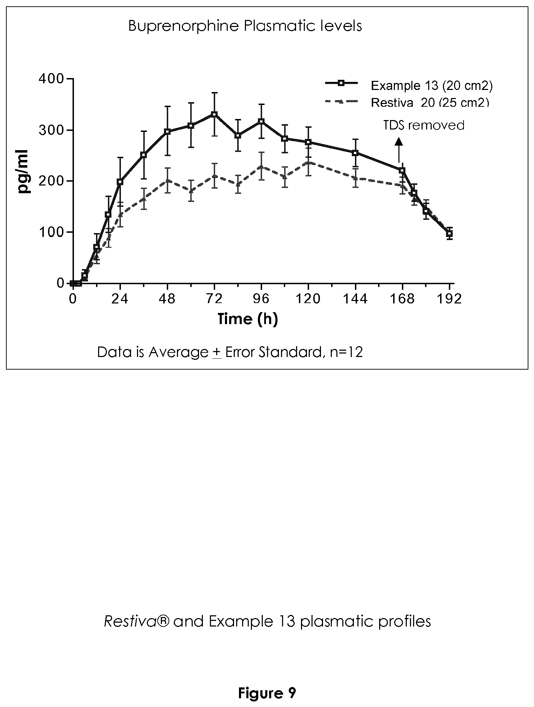

A pilot pharmacokinetics open randomised single-cross over (2 periods and 2 sequences) study was carried out on twelve (12) healthy individuals (volunteers) under in-patient conditions and Naltrexone block, in which it was compared the plasma levels reached by TDS with the composition detailed in Example 13 (with 20 cm² of active area) and the reference product Restiva^{®} TDS (with 25 cm² of active area). Results are shown in Figure 9.

The results of the study suggests that a TDS with the formulation of Example 13 and an active surface of 15.6 cm² theoretically would show an AUC equal to a TDS with Restiva^{®} having an active surface of 25 cm².

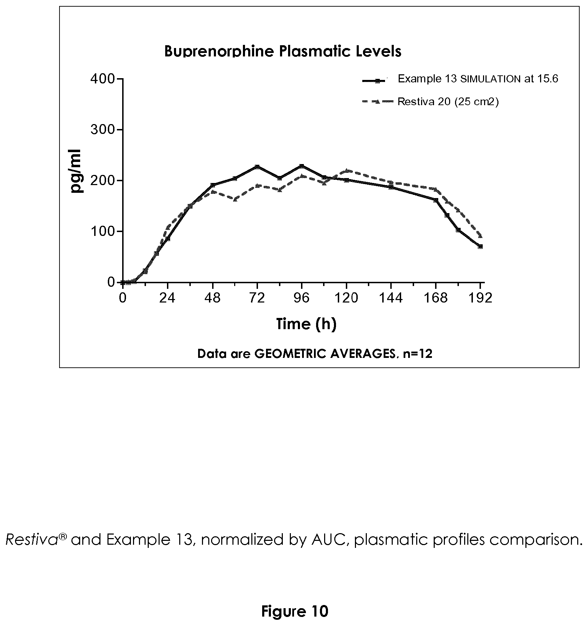

In Figure 10, it is shown that a simulation of the theoretical plasma profile reached by a TDS with the formulation of Example 13, but with an active surface of 15.6 cm², is quite similar to the one reached in the study by Restiva^{®} with an active surface of 25 cm².

### Examples 14 to 18:

The TDS with the composition detailed in Table 9 were produced.

**Table 9: Formulations of Examples 14 to 18**

| **Ingredient** | **Example 14 (BPF 343)** | **Example 15 (BPF 344)** | **Example 16 (BPF 345** | **Example 17 (BPF 346)** | **Example 18 (BPF 347)** |
|---|---|---|---|---|---|
| Buprenorphine Base | 8.0 | 8.0 | 8.0 | 8.0 | 8.0 |
| DT 87-4098 | 62.0 | 62.0 | 62.0 | 62.0 | 62.0 |
| Polyvinyl caprolactam, polyvinyl acetate and polyethylene glycol grafted copolymer, | --- | 2.5 | 5.0 | 7.5 | 10.0 |
| Levulinic acid | 10.0 | 10.0 | 10.0 | 10.0 | 10.0 |
| Isopropyl Myristate | 10.0 | 10.0 | 10.0 | 10.0 | 10.0 |

The TDS were stored at 40°C and 75% H.R. and at 25°C and 60% H.R. and were inspected regularly in order to find the presence of crystals of Buprenorphine.

The results of the observations are shown in Tables 10 and 11.

**Table 10: Presence of crystals in formulations stored at 40°C /75% H.R. (accelerated conditions)**

| **Presence of crystals** | | | | | | | |
|---|---|---|---|---|---|---|---|
| | Start | 7 days | 15 days | 30 days | 60 days | 90 days | 120 days |
| Example 14 | No | No | No | *Yes* | *Yes* | *Yes* | *Yes* |
| Example 15 | No | No | No | No | No | No | No |
| Example 16 | No | No | No | No | No | No | No |
| Example 17 | No | No | No | No | No | No | No |
| Example 18 | No | No | No | No | No | No | No |

**Table 11: Presence of crystals in formulations stored at 25°C /605% H.R. (accelerated conditions)**

| **Presence of crystals** | | | | | | | |
|---|---|---|---|---|---|---|---|
| | Start | 7 days | 15 days | 30 days | 60 days | 90 days | 120 days |
| Example 14 | No | No | No | No | No | *Yes* | *Yes* |
| Example 15 | No | No | No | No | No | No | No |
| Example 16 | No | No | No | No | No | No | No |
| Example 17 | No | No | No | No | No | No | No |
| Example 18 | No | No | No | No | No | No | No |

The results demonstrates that the polyvinyl caprolactam, polyvinyl acetate and polyethylene glycol grafted copolymer avoids the apparition of crystals acting as an inhibitor of crystallization along the time in accelerated and natural conditions of storage.

## Claims

1. A device for the transdermal administration of Buprenorphine, which comprises an adhesive matrix, a backing layer and release liner, where such adhesive matrix comprises a non-crosslinked adhesive acrylic polymer and the polymer(s) of the adhesive matrix is/are without carboxylic functionality, Buprenorphine Base, Levulinic Acid and Isopropyl Myristate, **characterised in that** the concentration of said Buprenorphine Base is smaller than or equal to 8% by weight of the total dry weight of said adhesive matrix, wherein the concentration of said Levulinic acid is from about 5% to about 10% by weight of the total dry weight of said adhesive matrix, wherein the concentration of said Isopropyl Myristate is from about 5% to about 10% by weight of the total dry weight of said adhesive matrix, and **in that** it has a total surface area of less than or equal to 23 cm².

2. A device for the transdermal administration of Buprenorphine, according to claim 1, **characterized in that** said non-crosslinked acrylic polymer adhesive without carboxylic functionality is a polymer with hydroxyl functionality.

3. A device for the transdermal administration of Buprenorphine, according to claim 1, **characterized in that** said non-crosslinked acrylic polymer adhesive without carboxylic functionality is a non-functional polymer.

4. A device for the transdermal administration of Buprenorphine according to any one of the preceding claims, **characterized by** further comprising a polyvinyl caprolactam, polyvinyl acetate and polyethylene glycol grafted copolymer.

5. A device for the transdermal administration of Buprenorphine according to claim 4, **characterized by** further comprising a polyvinyl caprolactam, polyvinyl acetate and polyethylene glycol grafted copolymer in a concentration from about 2.5% to about 10.0% by weight of the total dry weight of the adhesive matrix.

6. A device for the transdermal administration of Buprenorphine according to any one of claims 1 to 3, **characterized by** further comprising Polyvinylpyrrolidone.

7. A device for the transdermal administration of Buprenorphine according to claim 6, **characterized in that** the concentration of said Polyvinylpyrrolidone is from about 5% to about 10%, by weight of the total dry weight of the adhesive matrix.

8. A device for the transdermal administration of Buprenorphine according to claim 6 or claim 7, **characterized in that** said Polyvinylpyrrolidone has a K-value of 90.

## Patentansprüche

1. Vorrichtung für die transdermale Verabreichung von Buprenorphin, die eine Klebematrix, eine Trägerschicht und eine Abziehfolie umfasst, wobei solch eine Klebematrix einen nicht vernetzten Acrylpolymerklebstoff, wobei das/die Polymer(e) der Klebematrix ohne Carboxylfunktionalität ist/sind, Buprenorphin-Base, Lävulinsäure und Isopropylmyristat umfasst, **dadurch gekennzeichnet, dass** die Konzentration der Buprenorphin-Base geringer als oder gleich 8 Gew.-% des gesamten Trockengewichts der Klebematrix ist, wobei die Konzentration der Lävulinsäure etwa 5 bis etwa 10 Gew.-% des gesamten Trockengewichts der Klebematrix ist, wobei die Konzentration des Isopropylmyristats etwa 5 bis etwa 10 Gew.-% des gesamten Trockengewichts der Klebematrix ist, und dass sie eine Gesamtoberfläche von weniger als oder gleich 23 cm² aufweist.

2. Vorrichtung für die transdermale Verabreichung von Buprenorphin nach Anspruch 1, **dadurch gekennzeichnet, dass** der nicht vernetzte Acrylpolymerklebstoff ohne Carboxylfunktionalität ein Polymer mit Hydroxylfunktionalität ist.

3. Vorrichtung für die transdermale Verabreichung von Buprenorphin nach Anspruch 1, **dadurch gekennzeichnet, dass** der nicht vernetzte Acrylpolymerklebstoff ohne Carboxylfunktionalität ein nicht funktionelles Polymer ist.

4. Vorrichtung für die transdermale Verabreichung von Buprenorphin nach einem der vorangegangenen Ansprüche, **dadurch gekennzeichnet, dass** sie weiters ein Polyvinylcaprolactam-, Polyvinylacetat- und Polyethylenglykol-Pfropf-Copolymer umfasst.

5. Vorrichtung für die transdermale Verabreichung von Buprenorphin nach Anspruch 4, **dadurch gekennzeichnet, dass** sie weiters ein Polyvinylcaprolactam-, Polyvinylacetat- und Polyethylenglykol-Pfropf-Copolymer in einer Konzentration von etwa 2,5 bis 10,0 Gew.-% bezogen auf das gesamte Trockengewicht der Klebematrix umfasst.

6. Vorrichtung für die transdermale Verabreichung von Buprenorphin nach einem der Ansprüche 1 bis 3, **dadurch gekennzeichnet, dass** sie weiters Polyvinylpyrrolidon umfasst.

7. Vorrichtung für die transdermale Verabreichung von Buprenorphin nach Anspruch 6, **dadurch gekennzeichnet, dass** die Konzentration des Polyvinylpyrrolidons etwa 5 bis etwa 10 Gew.-% bezogen auf das gesamte Trockengewicht der Klebematrix beträgt.

8. Vorrichtung für die transdermale Verabreichung von Buprenorphin nach Anspruch 6 oder Anspruch 7, **dadurch gekennzeichnet, dass** das Polyvinylpyrrolidon einen K-Wert von 90 aufweist.

## Revendications

1. Dispositif pour l'administration transdermique de buprénorphine, qui comprend une matrice adhésive, une couche de support et un revêtement anti-adhésif, où une telle matrice adhésive comprend un polymère acrylique adhésif non réticulé et le ou les polymères de la matrice adhésive est/sont dépourvus de fonctionnalité carboxylique, de base de buprénorphine, d'acide lévulinique et de myristate d'isopropyle, **caractérisé en ce que** la concentration de ladite base de buprénorphine est inférieure ou égale à 8 % en poids du poids sec total de ladite matrice adhésive, dans lequel la concentration dudit acide lévulinique est d'environ 5 % à environ 10 % en poids du poids sec total de ladite matrice adhésive, dans lequel la concentration dudit myristate d'isopropyle est d'environ 5 % à environ 10 % en poids du poids sec total de ladite matrice adhésive, et **en ce qu'**il présente une superficie totale inférieure ou égale à 23 cm².

2. Dispositif pour l'administration transdermique de buprénorphine, selon la revendication 1, **caractérisé en ce que** ledit polymère acrylique adhésif non réticulé sans fonctionnalité carboxylique est un polymère avec une fonctionnalité hydroxyle.

3. Dispositif pour l'administration transdermique de buprénorphine, selon la revendication 1, **caractérisé en ce que** ledit polymère acrylique adhésif non réticulé sans fonctionnalité carboxylique est un polymère non fonctionnel.

4. Dispositif pour l'administration transdermique de buprénorphine selon l'une quelconque des revendications précédentes, **caractérisé en ce qu'**il comprend en outre un copolymère greffé de caprolactame de polyvinyle, d'acétate de polyvinyle et de polyéthylène glycol.

5. Dispositif pour l'administration transdermique de buprénorphine selon la revendication 4, **caractérisé en ce qu'**il comprend en outre un copolymère greffé de caprolactame de polyvinyle, d'acétate de polyvinyle et de polyéthylène glycol en une concentration d'environ 2,5 % à environ 10,0 % en poids du poids sec total de la matrice adhésive.

6. Dispositif pour l'administration transdermique de buprénorphine selon l'une quelconque des revendications 1 ou 3, **caractérisé en ce qu'**il comprend en outre de la polyvinylpyrrolidone.

7. Dispositif pour l'administration transdermique de buprénorphine selon la revendication 6, **caractérisé en ce que** la concentration de ladite polyvinylpyrrolidone est d'environ 5 % à environ 10 % en poids du poids sec total de la matrice adhésive.

8. Dispositif pour l'administration transdermique de buprénorphine selon la revendication 6 ou la revendication 7, **caractérisé en ce que** ladite polyvinylpyrrolidone présente une valeur K de 90.
